# EUROPEAN PATENT APPLICATION

(11) **EP 4 470 978 A1**
(43) Date of publication of application: **04.12.2024**
(21) Application number: 22923689.8
(22) Date of filing: 29.12.2022
(51) Int. Cl.: C02F 1/72

(54) **WATER/AIR TREATMENT SYSTEM FOR THE AGRICULTURAL AND INDUSTRIAL SECTORS**

(30) Priority: 25.01.2022 ES 202230110 U
(71) Applicant: Lopar Factory, S.L., 04746 La Mojonera - Almeria (ES)
(72) Inventor: PARRA JODAR, Marcos, 04720 ROQUETAS DE MAR (ALMERIA) (ES)
(74) Representative: Botella Reyna, Juan
(86) International application number: PCT/ES2022/070847
(87) International publication number: WO 2023/144428

(57) **Abstract**

The system is formed from a housing (1), wherein one or more water inlets and outlets (2) are established, and one or more treated air outlets (7), with means for regulating the flow of incoming water, and which has branches whereto a pneumatic injection unit (3) associated with one or more equipment (38)-(38b) generating oxidising and disinfecting gases is connected. The circuit has a non-return valve (9), overpressure safety valves (10), automatic and/or manual closing and opening means, water and airflow and pressure sensors (16), as well as one or more external connections (12) to take the air or gas of the adapted oxidation technology to the desired remote area to be treated, all with a control interface associated with one or more meters or electronic regulatory systems (17) of the amounts of air or gas to be injected into the system.

## Description

### OBJECT OF THE INVENTION

The present invention relates to a system that has been specially designed to allow the treatment of water/air without chemical products or reducing them, and that said water has specific properties to be used in several applications.

The invention is applicable in multiple areas, from the improvement of water qualities for: irrigation of plantations in agriculture, industrial washing of fruits, vegetables, processing material and packaging thereof (post-harvest), water treatment for swimming pools, disinfectant treatment in water from both domestic and industrial water treatment plants, its components such as reservoirs, filters, ducts, etc., dammed water, air treatment in food storage/refrigeration rooms, washing of clothes at an industrial level, etc.

The object of the invention is, therefore, to treat water and air, in order to maintain the same water, air and their derivatives, disinfected in a sustainable, healthy, ecological and efficient manner.

In addition to obtaining different additional benefits in the following areas:
In irrigation in agriculture;
- Reduction of phytosanitary chemical products,
- Improves the sustainability of agriculture and reduces environmental impact.
- Prevention of infections. Avoids the concentration of infectious agents, anaerobic organisms in water, soil and environment: bacteria, viruses, fungi. Function due to the oxidative reaction, so pathogenic microorganisms do not develop oxygen tolerance.
- Oxygenation of the root system.
- Improves soil quality by creating macro pores (spongy earth)
- Increased crop yield.
- Facilitates seed germination.
- Efficiency and homogeneity in irrigation. Clean the irrigation channels. Prevents the formation of residues in drippers, nebulisers, sprinklers and filters.
- Improvement of water and land quality since it degrades chlorine, chlorates, perchlorates, pesticides, herbicides that we can find in land and supply water.
- Reduces the growth of green algae, reducing infectious agents.

In post-harvest washing:
- Reduction of chemical products.
- Washing and disinfection of fruits, vegetables, processing material and packaging thereof in an ecological, effective, safe and healthy way.
- Safer and healthier food thanks to the reduction of chemical agents.
- It enhances sustainability by reducing environmental impact.

Swimming pool water:
- Reduction of chemical products for the maintenance of safe water.
- Prevents itching, redness and allergic skin reactions.
- Reduction of the emission of large volumes of water contaminated with chemical products to the drain.
- It enhances sustainability by reducing environmental impact.

Water from treatment plants/dammed or tank water:
a Reduction of green algae growth.
• Reduction of infectious agents.
• Reduction of chemical agents for treating
• It enhances sustainability by reducing environmental impact.

Food storage:
- Air treatment for a reduction of pathogens in the air and surfaces that accelerate the ripening, ageing and rotting process thereof.
- Create clean environments in food handling and treatment rooms, fruit and vegetable sector, winery sector and cork industry, etc.

Garments in the industrial sector:
- It disinfects garments with considerable energy savings, as these processes can be carried out with cold water, reducing emissions of volatile organic pollutants, as well as reducing the emission of large volumes of water contaminated with chemical products to the drain, offering economic savings in laundry/industrial cleaning products, all using technologies without the use of ozone, so harmful to health and for the conservation of certain equipment due to its corrosive power.

### BACKGROUND INFORMATION ON THE INVENTION

in the field of practical application of the invention, that of the devices that allow purifying/disinfecting water without using polluting chemical products, small equipment is known based on the use of oxidising/disinfecting gases used mainly in domestic washing machines, in order to avoid the use of detergents, treated water that is mixed with water from the mains in the water loading process of the washing machine.

More specifically, these equipment are based on the physical principle of heterogeneous photocatalysis whereby the cells of a reactor create a plasma that diffuses natural oxidants (superoxides, hydroxyls, hydroperoxides, hydrogen peroxide, among others). The natural oxidants that form the purifying plasma, surround the bacterial cells and the viruses, oxidising them and reacting with the cellular hydrogen and giving rise to the dehydration and lysing of the bacterial cells. This process is carried out by combining a high-density UV lamp and a catalyst (reactor) composed of a specific alloy of noble metals.

From an active cell, active photocatalytic oxidation is facilitated and the potential of ultraviolet light is maximised.

Photocatalytic oxidation allows producing one or more of the following oxidants (OX); hydroxyl radicals, vaporised hydrogen peroxide, superoxides, etc., which are used as sterilising gases for water, which are injected through a Venturi tube into a water treatment chamber.

This type of equipment has structural and functional limitations, such as: The technology they use composed of a high-density UV lamp and a catalyst (reactor) composed of a specific alloy of noble metals is located only inside the machine without it being possible to know if they are working correctly and as they are in its interior it makes maintenance and technical service very complex.

In some installations where more or less technology is required because more or less water must be treated, it is necessary to be able to increase or reduce the number of oxidation technology modules.

They cannot change, increase, reduce and adapt the model or intensity to the amount necessary for each installation independently of others.

This type of equipment can carry one or more water flow channels for independent treatment of the inlet water and Venturi injectors.

Venturi injectors work by depression in the water outlet part of the same. This means that at the inlet of the equipment you need to have more pressure than at the outlet.

If the water pressures in one or more venturis are equalised, they stop doing their job so they stop incorporating those gases into the water flow. This is very common in the vast majority of facilities where the incorporation of the equipment must be achieved.

This means that it is not possible to incorporate the water treatment equipment into the installation since it does not perform the function for which it was acquired.

On the other hand, installations often have to work with different flows and pressures when treating water for different sectors, for example, in irrigation in agriculture, since they have different sizes of farms with different levels of situation in each of the different irrigation sectors, with different dimensions, so this means that it only works in one or none of them.

In post-harvest washing machines, dammed water treatment, swimming pools and industrial clothes washing machines the same drawback occurs since in those facilities the water treatment system has to be installed in several units of machines or water distributions with different flow rates and pressures and connected in the different sectors of different pressure and flow rate.

At the same time, in many installations it is necessary to treat the water independently and without going through the machine, such as drums of stored water, dammed water that does not have access to the machine, pipes wherethrough the water passes but without going through the machine, etc.

In this way, as the water does not pass through the machine, it is impossible to treat it, so its incorporation becomes infeasible.

At other times the need is to treat the air in a food storage compartment such as refrigeration and storage chambers, etc.

In this type of equipment, the problem remains that for them to work they need the water to pass through them, so in this case it is also impossible to treat the air in the compartment.

### DESCRIPTION OF THE INVENTION

The system for the treatment of water that is recommended fills the aforementioned technical gap, being able to have an effective and stable application in multiple areas, from: the irrigation of plantations in agriculture both in place of installation where the water passes and remotely, industrial washing of fruits, vegetables, processing material and packaging thereof (post-harvest), water treatment for swimming pools, disinfectant treatment in water from both domestic and industrial water treatment plants, its components such as tanks, filters, pipes, etc., dammed water both in place of installation where the water passes and remotely, air treatment in food storage/refrigerated rooms both in place of installation and remotely, washing clothes industrially in one or more machines at the same time, etc.

To this end, and more specifically, the device of the invention is formed from an equipment wherein a housing participates, wherein one or more water inlets and one or more treated water outlets are established, with the special characteristic that the water inlet is divided into at least two branches, assisted at least one of these by valves/solenoid valves for adjusting the incoming water flow, branches whereto a pneumatic injection unit associated with one or more equipment for generating oxidising/disinfecting gases is connected, established externally to the housing, as well as optionally internally thereto if required, which are incorporated into the water flow system through extraction hoses connected to a distributor tube associated with a series of conduits with adapted connections.

In the housing, one or more outlet connections are established through injection outlet hoses with at least one non-return valve, having one or more overpressure safety valves, as well as at least one hose with automatic and/or manual closing and opening means, one or more water/air flow and pressure sensors, as well as one or more external connections to take the air/gas of the adapted oxidation technology to the desired remote area to be treated.

The housing has external connection fittings of the system up to the end point of incorporation of the air or gas of the adapted oxidation technology, as well as a control interface associated with one or more meters or electronic systems regulating the amounts of air or gas of the adapted oxidation technology to be injected into the system.

When the system is implemented in a post-harvest washing machine, clothes washing machine, irrigation for agriculture, or water purifier, the hose or hoses are connected to the pipes wherethrough the conventional water passes with collar-type connectors and adaptation terminations, where the manual closing and opening valve or valves or machined automatic solenoid valve are previously connected.

When the system is implemented in a reservoir or tank, the hose is introduced into the water to be treated, so that its end incorporates one or more air/gas diffusion/distribution nozzles of the oxidation technology adapted to that water, the control electronics of the device having means for water treatment in a continuous or timed manner.

When the system is implemented in compartments or rooms remote from the main housing, one or more nebulisation-type dosing nozzles and/or one or more distribution grilles of the type used in the ventilation air ducts are incorporated into the end of the hoses.

On the other hand, the means of measuring the air/gas flow to be injected into the system are capable of being arranged internally to the housing or adapted to an external control panel of the system.

This panel has built-in electrical differential and magneto-thermal protections, emergency stop and an automatic activation, stop and manual switch.

The panel additionally incorporates an analog and/or digital programmed activation mechanism which is managed through connection through Wi-Fi activation and mobile application.

According to another characteristic of the invention, one or more activation relays of the ON/OFF timed system are incorporated into the housing, which is activated through a button and one or more relays for the stop and start of the system during the work process.

The equipment has two or more internal fans which cool the internal mechanisms.

Air or gas extraction mechanisms of the adapted oxidation technology such as the mechanical venturis-type are optionally incorporated into the system pipes.

Finally, the housing includes an access door to its interior equipped with LED lighting.

### BRIEF DESCRIPTION OF THE DRAWINGS

To complete the description made below and aid a better understanding of the features of the invention, according to a preferred example of embodiment thereof, the description is accompanied by a set of drawings that form an integral part of same and which, for purposes of illustration only and in a non-limiting sense, show the following:

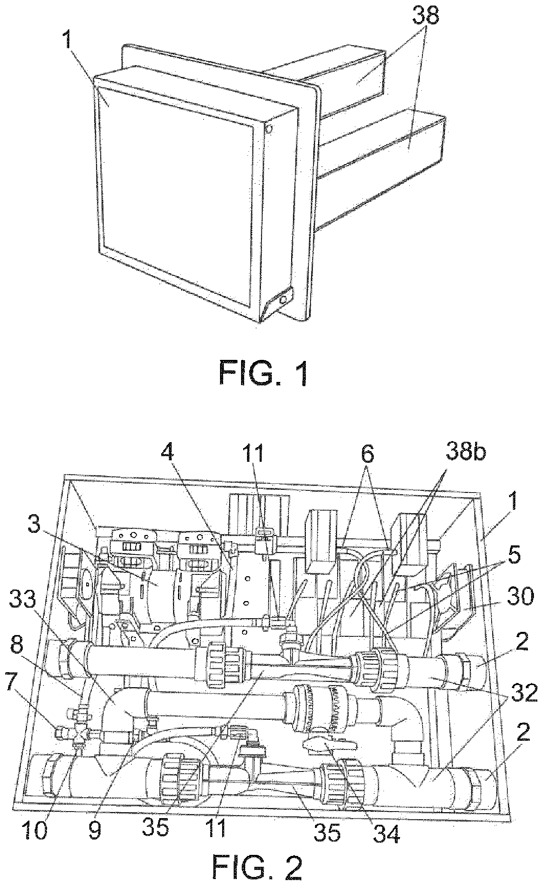

Figure 1.- Shows a general perspective view of a water-air treatment system made in accordance with the object of the present invention.
Figure 2.- Shows a perspective view of the internal structure contained in the housing of the main device of the system.

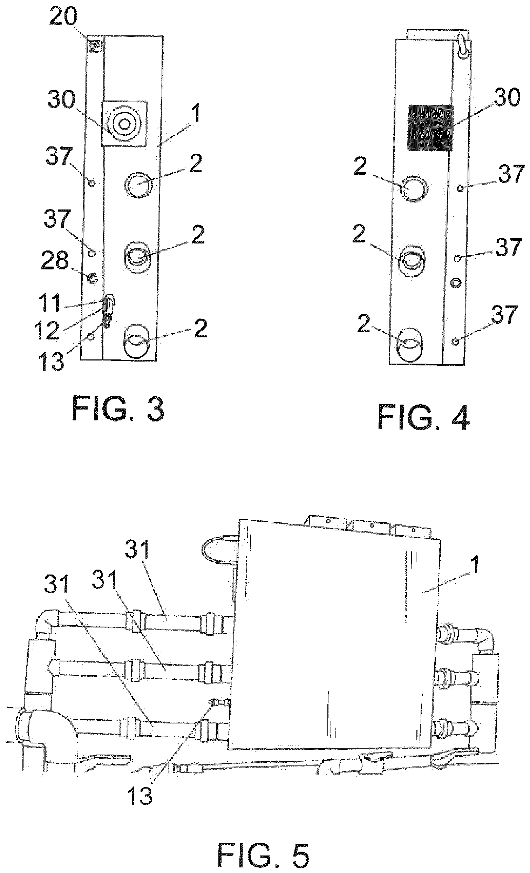

Figure 3.- Shows a side perspective view of the housing of figure 2.
Figure 4.- Shows a perspective view of the opposite side of the housing of figure 2.
Figure 5.- Shows a view of the external arrangement of the independent water or air treatment technology in the system of the invention.

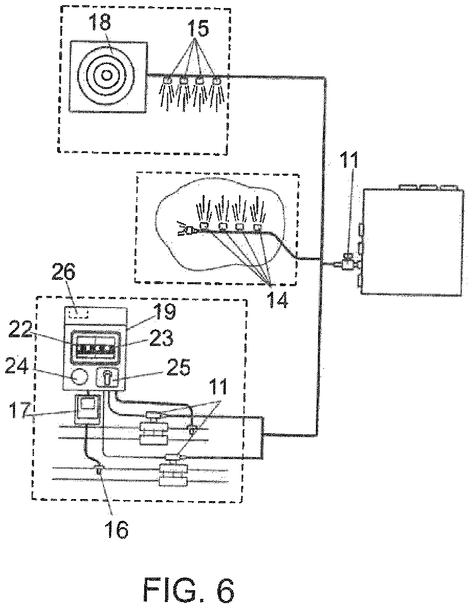

Figure 6.- Finally shows a schematic representation of the application of the invention in different areas such as remote air treatment, remote pipeline water treatment, and remote reservoir or tank treatment.

### PREFERRED EMBODIMENT OF THE INVENTION

The water and air treatment system adapts the independent technology or equipment (38)-(38b) generating oxidising/disinfecting gases, composed of a high-density UV lamp and a catalyst (reactor) composed of a specific alloy of noble metals or others.

It adapts it by incorporating this technology from the outside through the water orifices or inlets and outlets (2) provided for this purpose on the housing (1) so that the water and air treatment system becomes an independent adapter to the technology and intensity that you want to incorporate at any given time and according to the needs that are required depending on the installations or circumstances.

By incorporating it also from the outside, it is first possible to detect a fault or visual anomaly without having to open the machine, since these usually have LED fault warning indicators.

Thus, for technical maintenance or overhaul service, it is accessed from the outside without having to open the machine.

For the replacement of damaged components, greater possibility and accessibility is also achieved since it is performed from the outside.

This technology is composed of different models which vary in intensity, capacity, volume of air/air to be treated, etc. Being independent and adapting it to the device from the outside, it is possible to incorporate or replace it with the model that best suits the needs of the installation to be performed, which must on occasions be customised and adjusted to suit the needs of each customer.

At least one pneumatic injection unit (3) is established within the casing. This can be of the type: compressor or compressed air generator of any type (rolling piston-piston, rotating-rotating screw, membrane, scroll, centrifugal or turbo compressor, etc.) turbine, accumulator, etc.

Depending on the needs of the installation, at least one unit of one type or another is located in the system.

This automatically adjusts to the necessary pressure of the track of each sector in which it is working in order to be able to incorporate the gases generated by the oxidation technology adapted at any time and in any utility.

This injection extracts the gases formed by the adapted oxidation technology and incorporates them into the water flow system through extraction hoses (4) connected to a distributor tube associated with a series of conduits (5) with connections (6) adapted for this purpose.

One or more injector outlet connections (7) are incorporated in the outlet of the injector through injector outlet hoses (8) in at least one non-return valve (9) made of stainless steel or other anti-corrosive material in order to prevent the return of water to the air and gas injector of the adapted oxidation technology.

One or more overpressure safety valves (10) are incorporated with the function of opening automatically to avoid overpressure in the pipes and hoses if air/gas pressures sent by the injector are detected.

It is incorporated into the end of the hose, and may be one or more manual opening and closing valves (11) and/or one or more machined automatic opening and closing solenoid valve(s) inside and/or outside the system.

This solenoid valve may also have a manual and automatic adjustable opening agreed to allow a desired amount of air or gas to pass through, also incorporating one or more water or air flow and pressure sensors (16) to allow the air/gas of the necessary adapted oxidation technology to pass through depending on the utility, depending on the amount of water or air that is passing through.

One or more external connections (12) will be incorporated into the device to bring the air/gas of the adapted oxidation technology to the desired remote area to be treated.

This remote area is the one that for several reasons of impossibility that air or water cannot pass through the system. Either because it is a reservoir or water tank that is outside the installation area, or because what we want to treat is the air from an adjacent room or at a distance from where the system was installed or for comfort and flexibility in the installation.

It is connected to one or more sections of hose(s) to the external connection fittings (13) of the system to the end point where we want to incorporate the air/gas of the adapted oxidation technology.

If what we want to treat is a remote water flow to derive that air/gas from the oxidation technology adapted to a post-harvest washing machine, clothes washing machine, irrigation for agriculture, water purifier, etc., those hose(s) are connected to the pipes wherethrough the normal water passes with collar-type connectors and adaptation terminations, where the manual closing/opening valves or machined automatic solenoid valve are previously connected.

In the case of a reservoir, tank or similar, the system provides for the introduction of a section of that same hose or similar to that water to be treated. At the end of it, it may have one or more nozzles (14) for the diffusion and distribution of air or gas of the oxidation technology adapted to that water. The water can be treated continuously or timed as needed thanks to the system's control mechanisms.

For the environment of a remote room interior, the mechanism is the same with the difference that that air or gas of the adapted oxidation technology is diffused at the air level. At the end of the hoses, one or more nebulisation-type dosing nozzles (15) and/or one or more distribution grilles (18) of the ventilation air ducts-type or the like are incorporated.

In all these cases, the pneumatic injector(s) act to extract the air or gas from the adapted oxidation technology and drive it through the hoses connected to the external connection fittings to take it and incorporate it into that area, part or place to be treated.

The solenoid valves and sensors carry the connection of electrical wiring between themselves and one or more meters or regulating electronic systems (17), wherethrough the amounts of air or gas of the adapted oxidation technology that are to be established according to the needs can be regulated.

This meter or electronic system may be incorporated inside the system or outside by adapting it to the external control panel (19) of the system.

An external control panel (19) is incorporated which incorporates the electrical protections of differential (22) and magneto-thermal (23), emergency stop (24) and an automatic activation (25), stop and manual switch.

An analog and/or digital programmed activation mechanism is optionally incorporated, which is managed through connection through Wi-Fi activation (26) and mobile application.

In turn, the system incorporates an H-type branch connector (20) for quick connection to the M-type quick connector which connects the wiring that derives from the control panel which in turn connects to the electrical network of the installation.

It incorporates within the system one or more activation relays of the ON/OFF timed system, which is activated through a button (28) that also incorporates the system and one or more relays for the stop and start of the system during the work process for the rest of the electronic components and thus avoid overheating of the mechanisms.

In this way, the system can be activated in all possible ways, thus being applicable to any different sector, need and utility: timed controlled on and off, manual, stopped, automatic and spontaneous or programmed remote.

Two or more fans (30) are incorporated which cool the internal mechanisms such as the pneumatic injector(s), on the other hand the primary action is to introduce normal air and moisture from the outside on one side towards the inside of the system and expel it from the opposite side towards the outside of the system already treated by the adapted oxidation technology. The fans thus force the air and moisture through the oxidation technology chambers adapted in the system.

This action can be performed without the pneumatic injector being activated in order to have the environment of the room where the system is installed, clean and disinfected such as food handling and treatment rooms, storage rooms in the fruit and vegetable sector, rooms in the winery sector and cork industry, etc.

On the other hand, it incorporates gas-water mixers (31) of adapted oxidation technology on the outside of the system. These mechanisms mix the water that passes through the pipes incorporated into the system with the air/gas that is incorporated from the adapted oxidation technology that is incorporated into the water.

As many internal water flow pipes (32) as desired are incorporated into the system according to the amount of water that is wanted or required to be treated.

These pipes can be made of PVC material or similar depending on the pressure and flow requirements of the installation.

At least one of the pipes has a water flow branch (33) and regulation means (34) such as stopcocks or solenoid valves, with the function of venting and adapting the water flow rates in the installation.

Air or gas extraction mechanisms of the adapted oxidation technology such as the mechanical venturis (35) type are optionally incorporated into the system pipes.

These mixers are made of a translucent outer material of transparent PVC-type, reinforced with a withstand capacity of up to 10 bar and internal spirals. They also have built-in LED lighting that can be inside the pipe as well as outside. In turn, the LED lighting has a wiring with one or more M-type connector terminals to connect to the system. These light up automatically once the system is started.

This mechanism also allows us to verify the correct operation of the system since when the water mixed with air/gas bubbles that is incorporated from the adapted oxidation technology is seen to pass by, it gives rise to the verification of the correct operation by both the technical service and the end customer.

The system in turn incorporates one or more H-type connector terminals (37) to electrically power the LED lighting of the air or gas mixer(s) of the adapted oxidation technology.

Finally, the device incorporates LED lighting in the door for closing and opening access to its housing (1).

It is quickly verified that the system is in use thanks to this lighting.

## Claims

1. System for the treatment of water and air for the agricultural and industrial sector, which being of the type formed from a housing (1), wherein one or more water inlets and outlets (2) are established, where the water inlet is divided into at least one branch (33), assisted by at least one regulation means (34) for adjusting the incoming water flow, branches whereto a pneumatic injection unit (3) associated with one or more equipment (38)-(38b) generating oxidising gases is connected, is **characterised in that** it includes one or more treated air outlets (7), the equipment (38)-(38b) generating oxidising gases, established externally and/or internally to the housing (1), which are incorporated into the water treatment circuit through extraction hoses (4) connected to a distributor tube associated with a series of conduits (5) with and connections (6), it being provided that in the housing are established one or more outlet connections (7) through injector outlet hoses (8) in at least one non-return valve (9), having one or more overpressure safety valves (10), as well as at least one hose with automatic and/or manual closing and opening means, one or more water or air flow and pressure sensors (16), as well as one or more external connections (12) to take the air or gas of the oxidation technology to the desired remote area to be treated, the housing (1) having external connection fittings (13) of the system until the end point of incorporation of the air or gas of the oxidation technology, as well as a control interface associated with one or more meters or electronic systems regulators (17) of the amounts of air or gas of the oxidation technology to be injected into the system.

2. System for the treatment of water and air for the agricultural and industrial sector, according to claim 1, wherein the system is implemented in a post-harvest washing machine, clothes washing machine, irrigation for agriculture, or water purifier, wherein the hose or hoses are connected to the pipes wherethrough the water passes with coliar-type connectors and terminations where the manual closing and opening valve(s) or automatic machined solenoid valve is/are previously connected.

3. System for the treatment of water and air for the agricultural and industrial sector, according to claim 1, **characterised in that** the system is implemented in a reservoir or tank where a section of the hose is introduced into the water to be treated, whose end incorporates one or more nozzles (14) for the diffusion of air or gas of the oxidation technology adapted to that water, the control electronics of the device having means for water treatment in a continuous or timed manner.

4. System for the treatment of water and air for the agricultural and industrial sector, according to claim 1, **characterised in that** the system is implemented in compartments or rooms remote from the main housing (1), wherein at the end of the hose or hoses one or more nebulisation-type dosing nozzles (15) and/or one or more distribution grilles (18) of the ventilation air ducts-type are incorporated.

5. System for the treatment of water and air for the agricultural and industrial sector, according to claim 1, **characterised in that** the pneumatic injection unit (3) is of the compressor or compressed air generator-type.

6. System for the treatment of water and air for the agricultural and industrial sector, according to claim 1, **characterised in that** the means for measuring the air/gas flow to be injected into the system are arranged internally to the housing (1), or adapted to an external control panel (19) of the system.

7. System for the treatment of water and air for the agricultural and industrial sector, according to claim 6, **characterised in that** the external control panel (19) has electrical differential (22) and magneto-thermal (23) protections, emergency stop (24) and an automatic activation (25), stop and manual switch incorporated.

8. System for the treatment of water and air for the agricultural and industrial sector, according to claim 7, **characterised in that** the panel (19) incorporates an analog and/or digital programmed activation mechanism which is managed through a connection through Wi-Fi activation (26) and a mobile application,

9. System for the treatment of water and air for the agricultural and industrial sector, according to claim 1, **characterised in that** it incorporates an H-type branch connector (20) of quick connection electrical power supply with an M-type quick connector which connects the wiring that derives from the control panel that in turn connects to the electrical network of the installation.

10. System for the treatment of water and air for the agricultural and industrial sector, according to claim 1, wherein in the housing (1) one or more activation relays of the ON/OFF timed system are incorporated, which is activated through a button (28) and one or more relays for the stop and start of the system during the work process.

11. System for the treatment of water and air for the agricultural and industrial sector, according to claim 1, wherein two or more fans (30) are incorporated into the housing (1).

12. System for the treatment of water and air for the agricultural and industrial sector, according to claim 1, where the pipes of the system are incorporated with air or gas extraction mechanisms of the adapted oxidation technology such as mechanical venturis (35).

13. System for the treatment of water and air for the agricultural and industrial sector, according to claim 1, wherein the system Incorporates one or more H-type connector terminals (37) to electrically supply LED lighting implemented in the air or gas mixer or mixers of the adapted oxidation technology.

14. System for the treatment of water and air for the agricultural and industrial sector, according to claim 1, wherein the housing (1) includes an access door to its interior equipped with LED lighting.
